# NEUE EUROPÄISCHE PATENTSCHRIFT

(11) **EP 0 553 658 B2**
(45) Veröffentlichungstag und Bekanntmachung des Hinweises auf die Entscheidung über den Einspruch: **14.02.2007**
(45) Hinweis auf die Patenterteilung: 19.03.1997
(21) Anmeldenummer: 93100522.7
(22) Anmeldetag: 15.01.1993
(51) Int. Cl.: A61K 36/28

(54) **Pharmazeutisch wirksame Zusammensetzung aus Tanacetum parthenium sowie Verfahren zu deren Extraktion und mit der pharmazeutisch wirksamen Zusammensetzung hergestelltes Arzneimittel**
Pharmaceutically active composition from Tanacetum partenium, process of extraction and medicinal composition
Compositions pharmaceutiquement actives à de Tanacetum parthenium, procédé d'extraction et médicament

(30) Priorität: 31.01.1992 DE 4202657
(43) Veröffentlichungstag der Anmeldung: 04.08.1993
(73) Patentinhaber: SCHAPER & BRÜMMER GMBH & CO. KG, D-38259 Salzgitter (DE)
(72) Erfinder: Beuscher, Norbert, Dr., W-3320 Salzgitter 51 (DE); Willigmann, Ingo, Dr., W-3380 Goslar/Ohlof (DE)
(74) Vertreter: Lins, Edgar

(56) Entgegenhaltungen:
- EP-A- 0 065 106
- GB-A- 2 166 952
- JOURNAL OF CHROMATOGRAPHY Bd. 627 , 1992 , AMSTERDAM NL Seiten 255 - 261 ROGER M. SMITH ET AL. 'SUPERCRITICAL FLUID EXTRACTION AND GAS CHROMATOGRAPHIC DETERMINATION OF THE SESQUITERPENE LACTONE PARTHENOLIDE IN THE MEDICINAL HERB FEVERFEW (TANACETUM PARTHENIUM)'
- E. Stahl et al. "Verdichtete Gase zur Extraktion und Raffination", Springer Verlag Berlin, 1986, S. 47-62
- Römpps Chemie Lexikon, 7. Aufl. 1972-1977, S. 3186-3187
- E. Stahl et al., Perfumer & Flavorist 29, (1985) vol. 10, S. 29-37
- G. Brunner et al., Chem.-Ing.-Tech. (1981) vol. 53, S. 529-542
- Smith et al., J. Chromatography, vol. 627, S. 255-261 (1992)
- Smith et al., J. Chromatography, vol. 600, S. 175-181 (1992)

## Beschreibung

Die Erfindung betrifft ferner ein Verfahren zur Extraktion einer pharmazeutisch wirksamen Zusammensetzung aus feingemahlenern Tanacetum parthenium sowie ein mit der gewonnenen pharmazeutisch wirksamen Zusammensetzung hergestelltes Arzneimittel.

Für die Herstellung eines Extraktes aus der zerkleinerten Pflanze Tanacetum parthenium ist in der EP 0 098 041 A1 beschrieben, das Sesquiterpen-Lacton mit einem Öl aus der Pflanze zu extrahieren. Die Gewinnung des Sesquiterpen-Lactons aus der Pflanze erfolgt allgemein mit einem nichtpolaren organischen Lösungsmittel, wobei auch Leichtpetroleum, Hexan oder Chloroform in Frage kommen. Dabei kann nach der Extraktion mit einem ersten nichtpolaren Lösungsmittel dieses abgedampft werden und anschließend der Extrakt mit einem zweiten nichtpolaren Lösungsmittel als wenigstens erstes Eluat chromatographiert werden.

Aus einem Artikel "The Active Principle In Feverfew" in "The Lancet" vom 7. November 1981, Seite 1054, ist es bekannt, einen Extrakt aus der getrockneten Pflanze mit Leichtpetroleum herzustellen. Eine anschließende Chromatographie kann mit Benzol als apolarem Elutionsmittel durchgeführt werden. In "Biochemical Systematics and Ecology" 1977, Seiten 207 bis 218 ist die Extraktion von Sesquiterpen-Lactonen aus Tanacetum parthenium mit Chloroform, also einem apolaren Extraktionsmittel, beschrieben.

Bekannt ist ferner eine Extraktion mit einer Phosphatpuffer-Lösung durchzuführen.

Es hat sich gezeigt, daß die bekannten Extraktionsverfahren eine relativ geringe Ausbeute erbringen. Ferner erweisen sich die extrahierten Sesquiterpen-Lactone aus Tanacetum parthenium als relativ instabil.

Der Erfindung liegt die Aufgabe zugrunde, eine neue pharmazeutisch wirksame Zusammensetzung bzw. ein Arzneimittel aus Tanacetum parthenium zur Verfügung zu stellen, die eine höhere Stabilität aufweist.

Die pharmazeutisch wirksame Zusammensetzung aus Tanacetum parthenium mit Sesquiterpen-Lacton Parthenoliden als mengenmäßig dominierende Inhaltsstoffe ist durch folgende Verfahrensschritte erhältlich:
- Feinmahlen der Pflanze
- Durchführung einer Extraktion mit CO₂ im überkritischen Zustand bei einer Temperatur von 32° bis 60 °C und einem Druck von 150 bis 350 bar.

Die pharmazeutisch wirksame Zusammensetzung unterscheidet sich von den mit bisherigen Extraktionsverfahren gewonnenen Extrakten aus Tanacetum parthenium. Dies zeigt sich darin, daß der Parthenolidgehalt der erfindungsgemäßen Zusammensetzung über eine Lagerzeit von mehr als einem Jahr im wesentlichen konstant bleibt, während der Parthenolidgehalt herkömmlicher Extrakte über diesen Zeitraum drastisch abnimmt. Der Unterschied der erfindungsgemäßen pharmazeutisch wirksamen Zusammensetzung von den herkömmlichen Extrakten aus Tamacetum parthenium kann im wesentlichen darauf zurückgeführt werden, daß bei den herkömmlichen Verfahren charakteristische reaktive Gruppen der Sesquiterpen-Lactone, wie die exozyklische Methylengruppe und die Epoxidfunktion, zerstört worden sind, während sie in der erfindungsgemäßen Zusammensetzung erhalten geblieben sind.

Die obenerwähnte Aufgabe wird ferner gelöst durch ein Verfahren der eingangs erwähnten Art, bei dem die Extraktion mit CO₂ im überkritischen Zustand bei einer Temperatur von 32° bis 60 °C und einem Druck von 150 bis 350 bar durchgeführt wird.

Dieses Verfahren führt zu der obenerwähnten pharmazeutisch wirksamen Zusammensetzung mit den überraschenden Vorteilen.

Der mit dem erfindungsgemäßen Verfahren gewonnene Extrakt hat darüber hinaus den Vorteil, daß er frei von Restlösungsmitteln und darin eventuell enthaltenen nicht flüchtigen kontaminierenden Substanzen ist.

Die Extraktion mit überkritischem CO₂ erlaubt eine erschöpfende Extraktion der Droge, während dies mit anderen Lösungsmitteln nicht erreichbar ist.

Die Extraktion wird vorzugsweise bei einer Temperatur zwischen 32° und 60° C vorgenommen. Der bevorzugte Druckbereich liegt zwischen 150 und 350 bar, wobei bevorzugte Parameterkombinationen 60° C und 300 bis 350 bar oder 40° C und 200 bar sind.

Die erschöpfende Extraktion wird dadurch erreicht, daß das CO₂ im überkritischen Zustand zyklisch über die zerkleinerten Pflanzen geleitet wird. Im technischen Maßstab wird dabei eine Behandlungsdauer mit dem überkritischen Gas von etwa drei Stunden angewendet.

Die Extraktion von Tanacetum parthenium mit überkritischem CO₂ führt zu einem stabilen Extrakt, der reich an Sesquiterpen-Lactonen, insbesondere des Parthenolids ist. Die Extraktausbeute schwankt, je nach Wahl der Extraktionsbedingungen, zwischen 90 und 99 %. Die Parthenolidausbeute liegt entsprechend zwischen 11,9 und 14,4 %.

Der erfindungsgemäß hergestellte Extrakt läßt sich zur Herstellung eines Arzneimittels verwenden. Das Arzneimittel ist insbesondere zur Behandlung von Migräne, Asthma, Bronchitis oder Arthritis geeignet.

Es wurde überraschend gefunden, daß der an sich lipophile Extrakt noch einen Restwassergehalt von ca. 5 % haben kann. Dabei werden aus der Pflanze stammende polare Substanzen in den Extrakt verschleppt. Polare Inhaltsstoffe, u.a. Terpene und Chlorophylle, können bei der Formulierung des Arzneimittels mit Weichgelatinekapseln mit Aminogruppen der Gelatine reagieren und zu einer Quervernetzung führen, die eine rasche Auflösung der Gelatinehülle verhindert. Die für die Quervernetzung verantwortlichen Inhaltsstoffe sind nach Abschluß der Gerbung der Gelatinehülle nicht mehr im Kapselinhalt nachweisbar, sondern vollständig an die Kapselhülle gebunden. Dieser unerwünschte Effekt kann durch Zusatz von Kieselgel zur extraktionsbereiten Droge, vorzugsweise in Mengen von 60 bis 140 g pro kg feingemahlener Pflanze (Droge) verhindert werden. Eine optimale Zugabemenge beträgt 100 g pro kg feingemahlener Pflanze. Die Behandlung mit Kieselgel kann zu einer Abnahme der Extraktausbeute führen.

Es ist grundsätzlich bekannt, Extraktionen mit komprimierten Gasen durchzuführen. Eine Übersicht über die Grundlagen dieses Extraktionsverfahrens und der hauptsächlichen Anwendungsgebiete findet sich in Brunner, Peter "Zum Stand der Extraktion mit komprimierten Gasen" in Chem.-Ing.-Tech. 53 (1981) Seiten 529 bis 542. Neben der Darstellung der Grundlagen der Extraktion mit überkritischen Gasen geht aus Tabellen für Anwendungsbeispiele hervor, daß Hauptanwendungsgebiete bei der Gasextraktion bei Erdöl und ähnlichen Produkten, Kohle, Speiseölen und Fetten sowie bei der Entkoffeinierung von Kaffee bestehen. Ein einziger, nicht näher erläuterter Anwendungsfall für die Herstellung eines Pharmazeutikums ist genannt.

In dem Werk List, Schmidt "Technologie pflanzlicher Arzneizubereitungen", Stuttgart 1984, Seiten 159 bis 173 ist die Extraktion mit überkritischen Gasen ebenfalls erläutert. Als ein bevorzugtes Extraktionsgas ist dabei CO₂ genannt. Einige Anwendungen im pharmazeutischen Bereich sind aufgeführt. Als wesentlicher Vorteil wird angegeben, daß die Extrakte garantiert lösungsmittelfrei seien. Ein Hinweis darauf, daß durch die Extraktion mit CO₂ im überkritischen Zustand andere Extrakte als mit herkömmlichen Extraktionsverfahren erhalten werden und diese Extrakte andere Eigenschaften aufweisen, ist diesem Stand der Technik nicht zu entnehmen.

Die durch das erfindungsgemäße Extraktionsverfahren gewonnene pharmazeutisch wirksame Zusammensetzung enthält das Sesquiterpen-Lacton Parthenolid als mengenmäßig dominierenden Inhaltsstoff.

Die unter den bevorzugten Arbeitsbedingungen erzielten Extrakte haben eine fettartige Konsistenz, einen intensiven Geruch und werden mit zunehmender Extraktionsdauer dunkler. Der Extrakt wird vorzugsweise direkt in einem oder in einer Kombination von Ölen gelöst oder suspendiert, die üblicherweise in der Pharmazie verwendet werden. Die Lösung eignet sich insbesondere zum Einsatz von Weichgelatinekapseln. Andere Formulierungen sind jedoch auch möglich.

Der in der beschriebenen Weise gewonnene Extrakt zeigte in vergleichenden pharmakologischen Versuchen den anderen genannten Extraktionsformen überliegende Resultate in Bezug auf die antientzündliche und spasmolytische Eigenschaft.

Physikalisch-chemische Paramenter der Extraktion und des Extraktes sind in der nachfolgenden Tabelle 1 dargestellt

| Versuch | 2 | 3 |
|---|---|---|
| Extraktionsparameter | 350 bar/60° C | 200 bar/40° C |
| Farbe | dunkelolivgrün | olivgrün |
| Konsistenz | schmalzartig | schmalzartig |
| Geruch | arteigen aromatisch | arteigen aromatisch |
| Steigschmelzpunkt | 44 - 47° C | 43 - 47° C. |

Die nachstehend aufgeführten Beispiele erläutern die Gewinnung des Extraktes im technischen Maßstab:

### Beispiel 1

In einem 10-Liter-Extraktor, der mit allen Instrumenten zur Temperatur und Druckkontrolle ausgestattet war, wurden 3,2 kg fein gemahlene Droge von Tanacetum parthenium eingefüllt. Nach dem Verschließen der Apparatur wird aus dem Vorratstank gasförmiges CO₂ auf überkritische Werte gebracht, in die Anlage gefüllt und dann kontinuierlich im Kreislauf umgepumpt. Dieser Vorgang wiederholt sich zyklisch über einen Zeitraum von 3 Stunden bei 350 bar und 60° C, bis die Droge erschöpfend extrahiert ist.

Extraktausbeute 99,9 g; Parthenolidausbeute 11,4 g; Parthenolid-Gehalt des Extraktes 11,4 %; Parthenolid Restgehalt der extrahierten Droge 0,9 g.

### Beispiel 2

In einem 10-Liter-Extraktor, der mit allen Instrumenten zur Temperatur und Druckkontrolle ausgestattet war, wurden 3,1 kg fein gemahlene Droge von Tanacetum parthenium eingefüllt. Nach dem Verschließen der Apparatur wird aus dem Vorratstank gasförmiges CO₂ auf überkritische Werte gebracht, in die Anlage gefüllt und dann kontinuierlich im Kreislauf umgepumpt. Dieser Vorgang wiederholt sich zyklisch über einen Zeitraum von 3 Stunden bei 200 bar und 40° C, bis die Droge erschöpfend extrahiert ist.

Extraktausbeute 90,1 g; Parthenolid Ausbeute 11,9 g; Parthenolid-Gehalt des Extraktes 13,2 %; Parthenolid Restgehalt der extrahierten Droge 1,1 g.

Die Extraktion mit überkritischem CO₂ ergibt nicht nur eine wesentlich höhere Ausbeute für die Extrakte sondern auch eine wesentlich höhere Stabilität der Wirkstoffe. Die nachstehende Tabelle zeigt einen Vergleich zwischen einem herkömmlich wäßrig-ethanolischen Extrakt und einem erfindungsgemäßen CO₂ Extrakt anhand des Gehaltes an Parthenolid bei einer Lagerzeit von maximal etwa 1 1/2 Jahren.

Dabei zeigt sich ein erheblicher prozentualer Abfall des Parthenolidgehalts bei einem wäßrig-ethanolischen Extrakt, während der Parthenolidgehalt bei einem CO₂ Extrakt praktisch konstant bleibt.

| Lagerzeit (Tage) | wäßrig-ethanolischer Extrakt | | CO₂ -Extrakt | |
|---|---|---|---|---|
| | mg Parthenolid/g TR | % des Anfangswertes | mg Parthenolid/g TE | % des Anfangswertes |
| 0 | 8,26 | 100,0 | 119,7 | 100,0 |
| 5 | 5,81 | 70,3 | - | - |
| 7 | 7,48 | 90,6 | - | - |
| 8 | 5,60 | 67,8 | - | - |
| 20 | 3,08 | 37,3 | - | - |
| 26 | 2,34 | 28,3 | - | - |
| 32 | 1,92 | 23,2 | - | - |
| 35 | 1,63 | 19,7 | - | - |
| 47 | 1,39 | 16,8 | - | - |
| 54 | 1,22 | 14,8 | - | - |
| 126 | - | - | 111,3 | 93,0 |
| 132 | 1,05 | 12,7 | - | - |
| 313 | - | - | 116,9 | 97,7 |
| 576 | - | - | 117.2 | 97,9 |
| 587 | - | - | 123,5 | 103,2 |
| TR = Trockenrückstand | | | | |
| TE = Trockenextrakt | | | | |

## Patentansprüche

1. Verfahren zur Extraktion einer pharmazeutisch wirksamen Zusammensetzung aus feingemahlenern Tanacetum parthenium, **dadurch gekennzeichnet, daß** die Extraktion mit CO₂ im überkritischen Zustand bei einer Temperatur von 32° bis 60 °C und einem Druck von 150 bis 350 bar durchgeführt wird.

2. Verfahren nach Anspruch 1, **dadurch gekennzeichnet, daß** die Extraktion bei einer Temperatur von 60 °C und einem Druck von 300 bis 350 bar durchgeführt wird.

3. Verfahren nach Anspruch 2, **dadurch gekennzeichnet, daß** die Extraktion bei einer Temperatur von 40 °C und einem Druck von 200 bar durchgeführt wird.

4. Verfahren nach einem der Ansprüche 1 bis 3, **dadurch gekennzeichnet, daß** das CO₂ im überkritischen Zustand zyklisch über das feingemahlene Tanacetum parthenium geleitet wird.

5. Verfahren nach Anspruch 4, **dadurch gekennzeichnet, daß** die zyklische Umwälzung von CO₂ im überkritischen Zustand bis zur erschöpfenden Extraktion vorgenommen wird.

6. Verfahren nach einem der Ansprüche 1 bis 5, **dadurch gekennzeichnet, daß** der feingemahlenen Pflanze Tanacetum parthenium vor der Extraktion mit CO₂ Kieselgel hinzugefügt wird.

7. Verfahren nach Anspruch 6, **gekennzeichnet durch** die Zugabe von 60 bis 140 g Kieselgel pro kg feingemahlener Pflanze.

8. Verfahren nach Anspruch 7, **gekennzeichnet durch** die Zugabe von 100 g Kieselgel pro kg feingemahlener Pflanze.

9. Arzneimittel, enthaltend die nach dem Verfahren nach einem der Ansprüche 1 bis 8 gewonnene pharmazeutisch wirksame Zusammensetzung, die in einem pharmazeutisch verträglichen Öl gelöst oder suspendiert ist.

10. Arzneimittel nach Anspruch 9, **gekennzeichnet durch** die Darreichung in einer Weichgelatinekapsel.

## Claims

1. Process for extracting a pharmaceutically active composition from finely ground Tanacetum parthenium, **characterized in that** the extraction is carried out using CO₂ in the supercritical state, at a temperature of 32°C to 60°C and a pressure of 150 to 350 bar.

2. Process according to Claim 1, **characterized in that** the extraction is carried out at a temperature of 60°C and a pressure of 300 to 350 bar.

3. Process according to Claim 2, **characterized in that** the extraction is carried out at a temperature of 40°C and a pressure of 200 bar.

4. Process according to one of Claims 1 to 3, **characterized in that** the CO₂ in the supercritical state is fed cyclically over the finely ground Tanacetum parthenium.

5. Process according to Claim 4, **characterized in that** the cyclic circulation of CO₂ in the supercritical state is carried out until extraction is complete.

6. Process according to one of Claims 1 to 5, **characterized in that** silica gel is added to the finely ground plant Tanacetum parthenium before extraction using CO₂.

7. Process according to Claim 6, **characterized by** the addition of 60 to 140 g of silica gel per kg of finely ground plant.

8. Process according to Claim 7, **characterized by** the addition of 100 g of silica gel per kg of finely ground plant.

9. Medicament containing the pharmaceutically active composition which is obtained in accordance with the process according to one of Claims 1 to 8, dissolved or suspended in a pharmaceutically acceptable oil.

10. Medicament according to Claim 9, **characterized by** presentation in the form of a soft gelatin capsule.

## Revendications

1. Procédé d'extraction d'une composition à effet pharmaceutique à base de tanacetum parthenium trituré, **caractérisé en ce que** l'extraction est effectuée avec du CO₂ en état hypercritique à une température comprise entre 32 et 60 °C et avec une pression entre 150 et 350 bars.

2. Procédé selon la revendication 1, **caractérisé en ce que** l'extraction est effectuée à une température de 60 °C et avec une pression comprise entre 300 et 350 bars.

3. Procédé selon la revendication 2, **caractérisé en ce que** l'extraction est effectuée à une température de 40 °C et avec une pression de 200 bars.

4. Procédé selon l'une quelconque des revendications 1 à 3, **caractérisé en ce que** le CO₂ en état hypercritique est conduit de façon cyclique à travers le Tanacetum parthenium trituré.

5. Procédé selon la revendication 4, **caractérisé en ce que** la circulation cyclique du CO₂ en état hypercritique a lieu jusqu'à l'extraction exhaustive.

6. Procédé selon l'une quelconque des revendications 1 à 5, **caractérisé en ce que** du gel de silice est ajouté à la plante triturée Tanacetum parthenium avant l'extraction avec du CO₂.

7. Procédé selon la revendication 6, **caractérisé en ce que** 60 à 140 g de gel de silice sont ajoutés par kg de plante triturée.

8. Procédé selon la revendication 7, **caractérisé en ce que** 100 g de gel de silice sont ajoutés par kg de plante tritutrée.

9. Médicament contenant la composition à effet pharmaceutique obtenue par le procédé selon l'une quelconque des revendications 1 à 8, dissoute ou en suspension dans une huile pharmaceutiquement compatible.

10. Médicament selon la revendication 9, **caractérisé en ce qu'**il est présenté dans une capsule en gélatine souple.
